## Europäisches Patentamt

## European Patent Office

## Office européen des brevets

(19)

(11) Publication number: **0 053 824**
**B2**

# NEW EUROPEAN PATENT SPECIFICATION

(12)

(45) Date of publication of the new patent specification: 02.08.89

(51) Int. Cl.⁴: **C 07 C 65/11, C 07 C 51/15**

(21) Application number: 81110171.6

(22) Date of filing: 04.12.81

(54) Process for the production of 2-hydroxynaphthalene-6-carboxylic acid.

(30) Priority: 08.12.80 JP 171998/80

(43) Date of publication of application:
16.06.82 Bulletin 82/24

(45) Publication of the grant of the patent:
12.09.84 Bulletin 84/37

(45) Mention of the opposition decision:
02.08.89 Bulletin 89/31

(84) Designated Contracting States:
BE CH DE FR GB IT LI NL

(56) References cited:
WO-A-81/02573
DE-A-2 132 296
DE-A-2 449 779
DE-A-2 514 571
DE-A-2 837 053
DE-C-50 341
DE-C-436 524
US-A-1 503 984
US-A-1 593 816
US-A-2 131 357
US-A-2 132 356
US-A-2 824 892
US-A-3 405 169

Ullmanns Enzyklopädie der technischen
Chemie,3.Aufl.,Bd.13, pp.89,95 (1962)
Houben-Weyl, Bd. VIII. pp.373-374 (1952)

The file contains technical information submitted

(73) Proprietor: **Kabushiki Kaisha Ueno Seiyaku Oyo Kenkyujo, 2-31, Koraibashi, Higashi- ku Osaka-shi Osaka- fu (JP)**

(72) Inventor: **Ueno, Ryuzo, 10- 27, Nango- cho, Nishinomiya- shi Hyogo- ken (JP)**
Inventor: **Tsuchiya, Hiroaki, 251- 13, Tanabe, Motoyama- cho,Higashinada- ku, Kobe- shi Hyogo- ken (JP)**
Inventor: **Kuwae, Yoshihiko, 85, Miyauchi- cho 2-chome, Amagasaki- shi Hyogo- ken (JP)**
Inventor: **Muramoto, Yasukazu, 19, Ogino 4-chome, Itami- shi Hyogo- ken (JP)**

(74) Representative: **Glaeser, Joachim, Dipl.- Ing., Patentanwalt Flüggenstrasse 13, D-8000 München 19 (DE)**

(56) References cited: (continuation)
**after the application was filed and not included in this specification**

EP 0 053 824 B2

## Description

This invention relates to a process for producing 2-hydroxynaphthalene-6-carboxylic acid by the reaction of potassium beta-naphtholate with carbon dioxide.

2-Hydroxynaphthalene-6-carboxylic acid is an aromatic hydroxycarboxylic acid which has recently attracted attention as a material for polymers in combination with p-hydroxybenzoic acid because a polyester derived from this carboxylic acid and p-hydroxybenzoic acid gives fibers having high tensile strength and heat stability. Little, however, has been described in the literature about the method of synthesizing 2-hydroxynaphthalene-6-carboxylic acid, and to the best of the knowledges of the present inventors, U.S. Patent No. 1 593 816 to Andre et al. is the only pertinent reference. This U.S. Patent specification discloses a process for synthesizing 2-hydroxynaphthalene-6-carboxylc acid by the reaction of solid potassium beta-naphtholate with carbon dioxide. The U.S. Patent states that the reaction is carried out at 170 to 230°C for 8 hours, but is silent on the other reaction conditions, particularly the pressure of carbon dioxide and the proportion of by-product 2-hydroxynaphthalene-3-carboxylic acid. Furthermore, according to the process of the U.S. Patent, the final product is not sufficiently separated from the by-product acid or free beta-naphthol. Upon repeating the process of this U.S. Patent, the present inventors found that the yield of 2-hydroxynaphthalene-6-carboxylic acid is as low as less than 10 %, and the total yield of it and the by-product 2-hydroxynaphthalene-3-carboxylic acid is less than 30 % (see Referential Example given hereinbelow). Hence, this process is unsuitable for commercial production of 2-hydroxynaphthalene-6-carboxylic acid having such a purity as a material for polymers requires.

Furthermore in DE-A-2 837 053 there is described a process for the production of 2-hydroxynaphthalene-carboxylic acids by reaction of a liquid mixture of 1 mole of alkali beta-naphtholate, 0,05 to 3 moles of beta-naphthol and 0.1 to 5 times the weight of alkali betanaphtholate of gas oil and kerosene with carbon dioxide at a reaction temperature of at least 180°C and a carbon dioxide pressure of not more than 15 kg/cm2.G.

This process had been proposed by the inventors of the present invention. Later on the instant inventors studied a process by which to carry out the reaction of potassium beta-naphtholate with carbon dioxide in liquid state in order to establish a process for the industrial mass production of 2-hydroxynaphthalene-6-carboxylic acid. As a result, it has been found that if a specified liquid is used as the reaction medium, the mixture of this specified liquid and potassium beta-naphtholate becomes a liquid at reaction conditions and further, dilutes beta-naphthol liberated in the reaction system whereby favorable results are obtained for 2-hydroxynaphthalene-6-carboxylic acid yield and finishing treatment or the like.

It is an object of this invention therefore to provide a commercial process for producing 2-hydroxynaphthalene-6-carboxylid acid in quantities.

The present inventors investigated a process for reacting substantially anhydrous potassium betanaphtholate with carbon dioxide using a starting material mixture which is liquid under the reaction conditions employed. This work has led to the discovery that good results can be obtained in regard to the yield, work-up, etc. of 2-hydroxynaphthalene-6-carboxylic acid.

These advantages are obtainable when carrying out a process for the production of 2-hydroxynaphthalene-6-carboxylic acid which comprises changing potassium beta-naphtholate into a liquid mixture with an aliphatic, alicyclic or aromatic hydrocarbon or an aromatic ether having boiling points of 150 to 400°C under reaction conditions and reacting the liquid mixture with carbon dioxide at a temperature of 240 to 350°C and at a carbon dioxide pressure of 15 kg/cm2(G) or less.

The process of the present invention differs clearly from that of DE-A-2 837 053 in the respect that the reaction is carried out using a mixture of raw materials consisting entirely of potassium betanaphtholate and a reaction medium from the group of an aliphatic, alicyclic or aromatic hydrocarbon or an aromatic ether without addition of beta-naphthol. This reaction medium renders a mixture of it with potassium beta-naphtholate liquid under the reaction conditions, dilutes beta-naphthol which is formed during the reaction, and increases the proportion of 2-hydroxynaphthalene-6-carboxylic acid formed.

According to the process of this invention, 2-hydroxynaphthalene-6-carboxylic acid of high purity can be obtained in high yields by a simple and economical means. The yield of the product is about 40 % or higher which is more than twice as high as that obtained by the prior process cited above. The liquid reaction mixture obtained after the reaction is easy to work up, and the desired product and byproduct 2-hydroxynaphthalene-3-carboxylic acid which is also useful can be obtained in high purity, and the free beta-naphthol can be recovered almost completely. These favorable results are unexpected, and the present invention has, for the first time, established a commercial process for producing 2-hydroxynaphthalene-6-carboxylic acid of high purity which has an increasing demand in industry.

Examples of the reaction medium having a melting point of not more than 180°C used in a preferred embodiment of the process of this invention include aliphatic, alicyclic and aromatic hydrocarbons such as gasoline, kerosene, light oil (gas oil), lubricant oils, white oil, alkylbenzenes, alkylnaphthalenes, diphenyl and diphenylalkanes, and aromatic ethers such as diphenyl ether, anisole and ditolyl ether. They have a boiling point of 150 to 400°C. Petroleum- type hydrocarbons, especially light oil (gas oil) and kerosene, are preferred as the hydrocarbons.

Potassium beta-naphtholate used in the reaction can be prepared in a customary manner from beta-naphthol and potassium hydroxide. It should be sufficiently dehydrated for use in the invention.

According to the invention, the reaction between potassium beta-naphtholate and carbon dioxide is carried out at a temperature of 240 to 350°C, preferably 240 to 300°C, and a carbon dioxide pressure of at least 1 kg/cm$^2$.G preferably 1 to 15 kg/cm$^2$.G, more preferably at least 5.5 kg/cm$^2$.G, especially preferably 5.5 to 15 kg/cm$^2$.G. The starting mixture containing potassium beta-naphtholate should form a liquid system under the reaction conditions.

The reaction medium is used in an amount preferably 0.5 to 10 times, especially preferably 0.5 to 5 times, the weight of potassium beta-naphtholate. Larger amounts of the reaction medium may be used, but do not give better results in regard to the yield of the desired product, etc. From the standpoint of economy and work-up procedures, the upper limit of the amount of the reaction medium is preferably 10 times the weight of potassium beta-naphtholate. The reaction medium acts effectively to dilute free beta-naphthol formed in the liquid reaction system and to increase the proportion of 2-hydroxynaphthalene-6-carboxylic acid formed.

In a preferred embodiment of the invention, work-up is carried out as follows: Water is added to the reaction mixture after the reaction. The mixture is adjusted to pH 6.5 - 8 with an acid such as sulfuric acid or hydrochloric acid to free the unreacted potassium beta-naphtholate as beta-naphthol. When a reaction medium layer exists, it is separated before or after formation of free beta-naphthol. If required, a tarry layer containing beta-naphthol and resinous matter is separated in liquid form preferably by sedimentation at 80 to 100°C. The separated tarry layer is preferably washed with water, and the washing is reused as a portion of water to be added. Then, beta-naphthol is extracted from the water layer by using a hydrophobic extracting solvent which is preferably liquid at 110°C or lower.

Examples of suitable extracting solvents include hydrocarbons such as benzene, toluene, xylene, hexane and cyclohexane; halogenated hydrocarbons such as chlorobenzene, dichloromethane, dichloroethane and chloroform; nitrated hydrocarbons such as nitrobenzene and nitromethane; ethers such as dibutyl ether and diphenyl ether; ketones such as cyclohexanone, diisobutyl ketone and acetophenone; and alcohols having at least 4 carbon atoms such as n-butyl alcohol n-octyl alcohol and 2-ethylhexyl alcohol.

Preferably, the extraction is carried out by using the extracting solvent in an amount 0.3 to 2 times, especially 0.5 to 1.5 times, the volume of the water layer at a temperature of 30 to 110°C, especially 50 to 100°C. beta-Naphthol in the reaction medium layer can be recycled directly for reuse. Alternatively, beta-naphthol in the reaction medium layer and the extract may be recovered as an aqueous solution of potassium beta-naphtholate by reacting it with an aqueous solution of potassium hydroxide. The beta-naphthol in the tarry layer is recovered, for example, by vacuum distillation. The aqueous solution of potassium beta-naphtholate and the beta-naphthol which are thus recovered are recycled to a step of preparing the starting material and can be reused. By this extracting procedure, a trace of the reaction medium in the water layer can be removed, and the resinous matter does not substantially get mixed with the extract.

To recover the desired products, the pH of the water layer after the extraction is adjusted to 3.5 - 6, preferably 3.5 - 5.5, with an acid. Thus 2-hydroxynaphthalene-6-carboxylic acid precipitates selectively. Adjustment of the pH of the mother liquor to 1 - 3 preferably 1.5 - 2.5, results in the precipitation of a mixture of 2-hydroxynaphthalene-3-carboxylic acid and 2-hydroxynaphthalene-6-carboxylic acid. Separation of the mixture into the constituent acids can be effected, for example, by recrystallization from an organic solvent or a mixture of it with water.

The process of this invention can be performed batchwise or continuously.

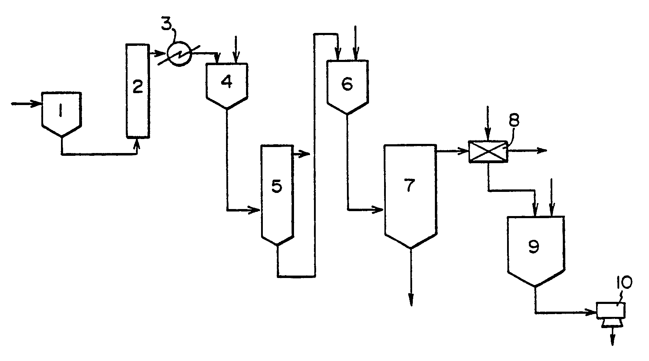

The continuous process is described specifically with reference to the accompanying drawing which is a diagram showing the steps of the process of this invention.

A mixture of potassium beta-naphtholate and a reaction medium stored in a storage tank 1 is sent to a reaction tank 2 where the starting mixture is reacted with carbon dioxide at the aforesaid temperature and carbon dioxide pressure. The residence time is preferably 2 to 6 hours. The reaction mixture from the reaction tank 2 is preferably cooled by a heat exchanger 3. Then, it is mixed with water in a water-mixing tank 4 with stirring. The mixture is then separated into a reaction medium layer and a water layer in a separating tank 5. Beta-naphthol can be recovered from the reaction medium layer (upper layer) by using a recovery device (not shown). Then, the water layer (lower layer) from the separating tank 5 is adjusted to pH 6.5 - 8 with an acid in a pH-adjusting tank 6 and sent to a separating tank 7 where the tarry layer is sedimented in liquid form. beta-Naphthol can be recovered from the tarry layer by using a vacuum distillation device (not shown), etc. The upper layer of the separating tank 7 is sent to an extracting device 8 (preferably a centrifugal extracting device) where it is extracted with a hydrophobic extracting solvent. From the solvent layer, beta-naphthol is recovered by using a recovery device (not shown). The water layer whose pH has been adjusted as above in the pH-adjusting tank 6 may be directly sent to the extracting device 8 without going through the separating tank 7. The water layer which has left the extracting device 8 is introduced into an acid precipitation tank 9 where acid precipitation is effected at a pH of 3.5 to 6 by adding an acid. The precipitate is then separated by a centrifugal separator 10. The crystals obtained from the centrifugal separator 10 are substantially pure 2-hydroxynaphthalene-6-carboxylic acid. By subjecting the mother liquor to acid precipitation in a separate acid precipitation tank (not shown) at a pH of 1 to 3, a mixture of 2-hydroxy-naphthalene-3-carboxylic acid and 2-hydroxynaphthalene-6-carboxylic acid is obtained, which can be fractionated by using an alcohol, etc.

The work-up step of the reaction mixture is advantageously carried out at a nearly constant temperature preferably in the range of 80 to 100°C, and for this purpose, an ordinary warming or heating device may be used.

The aqueous solution of potassium beta-naphtholate and beta-naphthol recovered from the reaction medium layer, the extracting solvent layer and the tarry layer are recycled to the step of preparing the starting material. If required, a part of beta-naphthol is recycled to the storage tank 1. The reaction medium and the extracting solvent which have been separated are recycled to the storage tank 1 and the extracting device 8 for reuse.

According to the process of this invention, the yield of 2-hydroxynaphthalene-6-carboxylic acid based on potassium beta-naphtholate can usually be 40 % or more by performing the reaction for 2 to 5 hours. The total yield of the products including 2-hydroxynaphthalene-3-carboxylic acid amounts to about 60 %. The ratio between the two acids can be properly adjusted. By using a relatively high reaction temperature or relatively low carbon dioxide pressure, the proportion of 2-hydroxynaphthalene-6-carboxylic acid can be increased. The total yield of the two acids based on the consumed beta-naphthol exceeds 85 %, and the ratio of recovery of beta-naphthol exceeds 85 %.

The reaction media which can be used in this invention are relatively inexpensive and have good properties. Moreover, free beta-naphthol formed during the reaction needs not to be removed, and can be recovered as above for reuse. The reaction medium and extracting solvent can be recycled for reuse without the need for subjecting them to a heating or cooling step, and this is quite advantageous for thermal economy. There are scarcely any losses of the reaction medium and the extracting solvent by degradation, etc., and the ratio of recovery of these materials is more than 99.5 %. The process of this invention is also advantageous from the viewpoint of thermal economy because the separation of the reaction medium layer, the separation of the tarry layer and the extraction of the water layer after the reaction can be carried out at substantially the same temperature. In addition, according to this invention, all process steps for producing 2-hydroxynaphthalene-6-carboxylic acid from beta-naphthol can be practiced continuously. Hence, the present invention provides a process which is very advantageous commercially.

The following non-limitative examples illustrate the present invention more specifically.

**Referential Example**

According to what should be the best conditions of the process (solid phase process) described in U.S. Patent No. 1 593 816, 182 g of solid potassium beta-naphtholate was reacted with carbon dioxide in a stirred autoclave at a temperature of 230°C and a carbon dioxide pressure of 4 kg/cm²6.G for a period of 8 hours. There were only obtained 18.4 g (yield 9.8 % based on potassium beta-naphtholate) of 2-hydroxynaphthalene-6-carboxylic acid and 34.2 g (yield 18.2 % based on potassium beta-naphtholate) of 2-hydroxynaphthalene-3-carboxylic acid. (The two acids were analyzed by gas-chromatography after esterifying them with diazomethane).

**Example 1**

A pressure reaction kettle was charged with 364 g of light oil (gas oil having a boiling range of 200 to 310°C) and 182 g of potassium beta-naphtholate. With stirring, potassium beta-naphtholate was reacted with carbon dioxide at a temperature of 260°C and a carbon dioxide pressure of 6 kg/cm².G for a period of 4 hours.

The reaction mixture was added to 830 ml of water, and the mixture was heated at 100°C for 30 minutes to decompose the by-product 2-hydroxynaphthalene-1-carboxylic acid salt. The mixture was separated at 85°C into a light oil layer and a water layer. The water layer was adjusted to pH 7.0 with dilute sulfuric acid, and the tarry layer sedimented was separated at the same temperature. The water layer was extracted with 500 ml of toluene at 80°C. Then, the water layer was adjusted to pH 4.5 with dilute sulfuric acid and cooled to room temperature, whereupon 71.4 g of 2-hydroxy-naphthalene-6-carboxylic acid was obtained as crystals. The mother liquor after separation of the crystals was adjusted to pH 2.0 with dilute sulfuric acid to give 18.8 g of crystals containing 3.8 g of 2-hydroxynaphthalene-6-carboxylic acid and 15.0 g of 2-hydroxynaphthalene-3-carboxylic acid (the contents of the two acids were measured by esterifying them with diazomethane and analyzing the esters by gas-chromatography). The mixture was fractionally recrystallized from dilute methanol to give the respective acids in pure form.

The yield of 2-hydroxynaphthalene-6-carboxylic acid based on potassium beta-naphtholate was 40.0 % and the total yield of the two acids on the same basis was 48.0 %. beta- Naphthol was recovered in a total amount of 65.5 g from the light oil layer, the tarry layer and the toluene layer. The total yield of the two acids based on the consumed beta-naphthol was 88.1 %, and the ratio of recovery of betanaphthol was 87.5 %.

## Example 2

The procedure of Example 1 was followed except that 273 g of 1-phenyl-1-(2,3-dimethyl phenyl)ethane having a boiling range of 292 to 306°C was used instead of the light oil in Example 1. There were obtained 65.0 g of 2-hydroxynaphthalene-6-carboxylic acid and 16.2 g of 2-hydroxynaphthalene-3-carboxylic acid, and 69.5 g of beta-naphthol was recovered. The yield of 2-hydroxynaphthalene-6-carboxylic acid based on potassium beta-naphtholate was 34.6 %, and the total yield of the two acids on the same basis was 43.2 %. The total yield of the two acids based on the consumed beta-naphthol was 83.6 %, and the ratio of recovery of beta-naphthol was 85.0 %.

## Example 3

The procedure of Example 1 was followed except that 546 g of kerosene having a boiling range of 180 to 280°C was used instead of the light oil in Example 1. There were obtained 73.2 g of 2-hydroxynaphthalene-6-carboxylic acid and 14.3 g of 2-hydroxynaphthalene-3-carboxylic acid, and 67.0 g of beta-naphthol was recovered. The yield of 2-hydroxynaphthalene-6-carboxylic acid based on potassium beta-naphtholate was 38.9 %, and the total yield of the two acids was 46.5 %. The total yield of the two acids based on the consumed beta-naphthol was 86.9 %, and the ratio of recovery of beta-naphthol was 86.9 %.

## Example 4

The procedure of Example 1 was followed except that 546 g of Dowtherm (a mixture of 75 % of diphenyl ether and 25 % of diphenyl) was used instead of the light oil in Example 1. There were obtained 64.7 g of 2-hydroxynaphthalene-6-carboxylic acid and 16.3 g of 2-hydroxynaphthalene-3-carboxylic acid, and 69.6 g of beta-naphthol was recovered. The yield of 2-hydroxynaphthalene-6-carboxylic acid based on potassium beta-naphtholate was 34.4 %, and the total yield of the two acids on the same basis was 43.1 %. The total yield of the two acids based on the consumed beta-naphthol was 83.3 %, and the ratio of recovery of beta-naphthol was 84.9 %.

## Example 5

By using the device shown in the accompanying drawing, the reaction and work-up in accordance with this invention were continuously carried out.

A liquid dispersed mixture composed of anhydrous potassium beta-naphtholate and light oil (gas oil) in a weight ratio of 91 : 182 was put in the storage tank 1. The mixture was sent in an amount of 273 kg per hour to reaction tank 2 kept at a carbon dioxide pressure of 6 km/cm$^2$.G, and reacted with carbon dioxide at 260°C. The residence time was 4 hours. The reaction mixture which left the reaction tank 2 was cooled by heat exchanger 3, and mixed in water-mixing tank 4 with 400 liters/hr of water with stirring. The temperature of the mixture was adjusted to 85°C, and the mixture was then introduced into separating tank 5 where it was separated at 85°C into a light oil layer and a water layer. From the upper light oil layer, beta-naphthol was recovered by using a recovery device (not shown). The lower water layer was adjusted to pH 7.0 with dilute sulfuric acid in pH-adjusting tank 6, and then separated at 85°C in separating tank 7. The lower tarry layer separated in the separating tank 7 was subjected to a vacuum distillation device (not shown) to recover beta-naphthol. The upper layer in the separating tank 7 was sent to a centrifugal extracting device 8 wherein it was extracted at 85°C with 250 liters/hr of xylene. The xylene layer was sent to a recovery device (not shown) to recover naphthol. The water layer which left the extracting device 8 was sent to acid precipitation tank 9 where it was subjected to acid precipitation at a pH of 4.5, and the precipitate was separated by centrifugal separator 10. There was obtained 35.8 kg/hr of 2-hydroxynaphthalene-6-carboxylic acid as crystals. The mother liquor left after the centrifugal separation was adjusted to pH 2.0 with dilute sulfuric acid in a separate acid precipitation device (not shown) to give hourly 9.5 kg of crystals containing 1.9 kg of 2-hydroxynaphthalene-6-carboxylic acid and 7.6 kg of 2-hydroxynaphthalene-3-carboxylic acid. The mixture was fractionally recrystallized from dilute methanol by a recrystallization device (not shown) to give the respective acids nn pure form.

The yield of 2-hydroxynaphthalene-6-carboxylic acid based on potassium beta-naphtholate was 40.1 %, and the total yield of the two acids was 48.2 %. 32.6 kg of beta-naphthol was recovered hourly. The total yield of the two acids based on the consumed beta-naphtholate was 88.1 %, and the ratio of recovery of beta-naphthol was 87.5 %.

## Example 6

By using the device shown in the accompanying drawing, the reaction and work-up were carried out in accordance with this invention were carried out continuously.

A liquid dispersed mixture composed of anhydrous potassium beta-naphtholate and light oil (gas oil) in a weight ratio of 91 : 182 was stored in storage tank 1. The mixture was sent in an amount of 273 kg per hour to reaction tank 2 kept at a carbon dioxide pressure of 9 kg/cm$^2$.G, and reacted with carbon dioxide at 280°C. The residence time was 3 hours. The reaction mixture which left the reaction tank 2 was cooled by heat exchanger 3, and mixed with 400 liters/hr of water with stirring in water-mixing tank 4. The temperature of the mixture was adjusted to 85°C, and the mixture was then introduced into separating tank 5 where it was separated at 85°C into a light oil layer and a water layer. From the upper light oil layer, beta-naphthol was recovered by using a recovery device (not shown). The lower water layer was adjusted to pH 7.0 with dilute sulfuric acid in pH-adjusting tank 6, and then without going through separating tank 7, was directly sent to centrifugal extracting device 8 where it was extracted at 85°C with 300 ml/hr of xylene. The xylene layer was sent to a recovery device (not shown) to recover beta-naphthol. The water layer which left the extraction device 8 was sent to acid precipitation tank 9 where it was subjected to acid precipitation with dilute sulfuric acid at a pH of 4.2. The precipitate was separated by centrifugal separator 10. There was obtained hourly 35.3 kg of 2-hydroxynaphthalene-6-carboxylic acid as crystals. The mother liquor left after the centrifugal separation was adjusted to pH 2.0 with dilute sulfuric acid in a separate acid precipitation device (not shown) to give 4.7 kg of crystals consisting of 1.5 kg of 2-hydroxynaphthalene-6-carboxylic acid and 3.2 kg of 2-hydroxynaphthalene-3-carboxylic acid. The mixture was fractionally recrystallized from dilute methanol in a recrystallization device (not shown) to give the respective acids in pure form.

The yield of 2-hydroxynaphthalene-6-carboxylic acid based on potassium beta-naphtholate was 39.1 %, and the total yield of the two acids on the same basis was 42.6 %. 36.8 kg of beta-naphthol was recovered. The total yield of the two acids based on the consumed beta-naphthol was 87.1 %, and the ratio of recovery of beta-naphthol was 87.3 %.

## Claims

1. A process for the production of 2-hydroxynapnthalene-6-carboxylic acid which comprises changing potassium beta-naphtholate into a liquid mixture with an aliphatic, alicyclic or aromatic hydrocarbon or an aromatic ether having boiling points of 150 to 400°C under reaction conditions and reacting the liquid mixture with carbon dioxide at a temperature of 240 to 350°C and at a carbon dioxide pressure of 15 kg/cm$^2$(G) or less.

2. A process as set forth in claim 1 wherein the aliphatic, alicyclic or aromatic hydrocarbon having boiling points of 150 to 400°C is a hydrocarbon of the petroleum series.

3. A process as set forth in claim 2 wherein the hydrocarbon of the petroleum series is light oil or kerosene.

4. A process as set forth in claim 1 wherein the aliphatic, alicyclic or aromatic hydrocarbon or aromatic ether is used in an amount of 0.5 to 10 times the weight of potassium beta-naphtholate.

5. A process as set forth in claim 1 wherein water is added to a reaction mixture after completion of reaction, a layer of the aliphatic, alicyclic or aromatic hydrocarbon or aromatic ether having boiling points of 150 to 400°C is separated; a tarry layer is precipitated in a liquid state from the aqueous layer and separated; beta-naphthol is extracted from the aqueous layer using a hydrophobic extracting solvent which is in a liquid state at 110°C or below; and the aqueous layer left over after the extracting is subjected to acid precipitation.

## Patentansprüche

1. Verfahren zur Herstellung von 2-Hydroxynaphthalin-6-carbonsäure, dadurch gekennzeichnet, daß man Kaliumbeta-naphtholat mit einem aliphatischen, alicyclischen oder aromatischen Kohlenwasserstoff oder einem aromatischen Äther mit Siedepunkten von 150 bis 400°C unter Reaktionsbedingungen in ein flüssiges Gemisch überführt und das flüssige Gemisch mit Kohlendioxid bei einer Temperatur von 240 bis 350°C und bei einem Kohlendioxidüberdruck von 15 kg/cm$^2$ oder weniger umsetzt.

2. Verfahren nach Anspruch 1, dadurch gekennzeichnet, daß der aliphatische, alicyclische oder aromatische Kohlenwasserstoff mit einem Siedepunkt von 150 bis 400°C ein Kohlenwasserstoff der Erdölreihe ist.

3. Verfahren nach Anspruch 2, dadurch gekennzeichnet, daß der Kohlenwasserstoff der Erdölreihe Leichtöl oder Kerosin ist.

4. Verfahren nach Anspruch 1, dadurch gekennzeichnet, daß man den aliphatischen, alicyclischen oder aromatischen Kohlenwasserstoff oder aromatischen Äther in einer Menge des 0,5- bis 10-fachen des Gewichts von Kalium-betanaphtholat verwendet.

5. Verfahren nach Anspruch 1, dadurch gekennzeichnet, daß man Wasser zu einer Reaktionsmischung nach Vervollständigung der Umsetzung zusetzt, eine Schicht aus aliphatischem, alicyclischem oder aromatischem Kohlenwasserstoff oder aromatischem Äther mit einem Siedepunkt von 150 bis 400°C abtrennt, eine teerartige Schicht in flüssigem Zustand aus der wäßrigen Schicht ausfällt und abtrennt, β-Naphthol aus der wäßrigen Schicht unter Verwendung eines hydrophoben Extraktionslösungsmittels, das bei 110°C oder darunter in flüssigem Zustand vorliegt, extrahiert, und die nach der Extraktion zurückbleibende wäßrige Schicht einer Säureausfällung unterwirft.

## Revendications

1. Un procédé de production de l'acide 2-hydroxynaphtalène-6-carboxylique selon lequel on forme un mélange liquide avec du bêta-naphtolate de potassium et un hydrocarbure aliphatique, alicyclique ou aromatique ou un éther aromatique, ayant des points d'ébullition de 150 à 400°C dans les conditions de la réaction, mélange liquide que l'on fait réagir avec du dioxide de carbone à une température de 240 à 350°C sous une pression relative du gaz carbonique de 15 bars ou moins.

2. Procédé selon la revendication 1 dans lequel l'hydrocarbure aliphatique, alicyclique ou aromatique à point d'ébullition de 150 à 400°C est un hydrocarbure de pétrole.

3. Procédé selon la revendication 2 dans lequel l'hydrocarbure de pétrole est une huile légère (gazole) ou du kérosène.

4, Procédé selon l'une quelconque des revendications 1 à 3 dans lequel l'hydrocarbure aliphatique, alicyclique ou aromatique ou l'éther aromatique est ajouté dans une proportion de 0.5 à 10 fois le poids du bêta-naphtolate de potassium.

5. Procédé selon l'une quelconque des revendications 1 à 4 dans lequel on ajoute de l'eau au mélange réactionnel après la fin de la réaction et on sépare la couche de l'hydrocarbure aliphatique, alicyclique ou aromatique ou de l'éther aromatique à point d'ébullition de 150 à 400°C; de la couche aqueuse on fait précipiter une couche goudronneuse à l'état liquide et on la sépare; on extrait le bêta-naphtol de la couche aqueuse avec un solvant hydrophobe qui est à l'état liquide à la température de 110°C ou moins; et après cette extraction on fait précipiter l'acide formé de la couche aqueuse restante.